# EUROPÄISCHE PATENTSCHRIFT

(11) **EP 1 538 140 B1**
(45) Veröffentlichungstag und Bekanntmachung des Hinweises auf die Patenterteilung: **04.02.2009**
(21) Anmeldenummer: 04026130.7
(22) Anmeldetag: 04.11.2004
(51) Int. Cl.: C07C 59/68, C11D 1/74

(54) **Ethercarbonsäuren auf Basis alkoxylierter Styrylphenole**
Ether carboxylic acids based on alkoxylated styrylphenol
Acides carboxyliques d'éther à base de styrylphenol alcoxylé

(30) Priorität: 17.11.2003 DE 10353603
(43) Veröffentlichungstag der Anmeldung: 08.06.2005
(73) Patentinhaber: Clariant Produkte (Deutschland) GmbH, 65929 Frankfurt am Main (DE)
(72) Erfinder: Dahlmann, Uwe, Dr., 69126 Heidelberg (DE); Kupfer, Rainer, Dr., 65795 Hattersheim (DE)

(56) Entgegenhaltungen:
- EP-A- 1 354 905
- WO-A-02/092583
- US-B1- 6 326 514
- PATENT ABSTRACTS OF JAPAN Bd. 014, Nr. 403 (C-0753), 31. August 1990 (1990-08-31) & JP 02 151619 A (TORAY IND INC), 11. Juni 1990 (1990-06-11)
- PATENT ABSTRACTS OF JAPAN Bd. 1999, Nr. 01, 29. Januar 1999 (1999-01-29) & JP 10 270846 A (EBARA YUUJIRAITO KK), 9. Oktober 1998 (1998-10-09)

## Beschreibung

Die vorliegende Erfindung betrifft die Verwendung von Ethercarbonsäuren auf Basis alkoxylierter Styrylphenole, als oberflächenaktive Additive.

Ethercarbonsäuren, d.h. organische Carbonsäuren, die neben der Carboxylfunktion eine oder mehrere Etherbrücken tragen, bzw. deren Alkali- oder Aminsalze, sind als milde nichtionische bzw. anionische Detergenzien mit hohem Kalkseifendispergiervermögen bekannt. Sie finden sowohl in Waschmittel- und Kosmetikformulierungen, aber auch in technischen Anwendungen (z.B. Metallbearbeitungsflüssigkeiten, Kühlschmiermittel, gewerbliche Reiniger, Additive für Textil- und Lederverarbeitung, Hilfsmittel bei der Papier- und Zellstoffherstellung) Verwendung.

Diese Produkte werden gemäß dem Stand der Technik entweder durch Alkylierung von Alkohol- oder Fettalkoholoxethylaten oder -oxpropylaten mit Chloressigsäurederivaten (Williamsonsche Ethersynthese) oder aus den gleichen Ausgangsprodukten durch Oxidation mit verschiedenen Reagenzien (Luftsauerstoff, Hypochlorit, Chlorit) unter Katalyse mit verschiedenen Katalysatoren hergestellt.

DE-C-199 28 128 offenbart ein Verfahren zur Herstellung von Ethercarbonsäuren mit niedrigem Restalkoholgehalt, indem Fettalkohole zunächst unter Einsatz nichtkatalytischer Mengen an Alkalikatalysator (NaOH, KOH, Alkoholate über 5 Mol-%) mit Alkylenoxiden umgesetzt werden, und die resultierenden hochalkalischen Reaktionsmischungen, die aus einem Gemisch von oxethylierten Alkoholen und Alkoholaten verschiedener Polyalkylenglykolether bestehen, anschließend in einer klassischen Williamson-Synthese mit Natriumchloracetat in die entsprechende Ethercarbonsäure überführt werden. Hierdurch wird der Restgehalt an Fettalkohol in der Ethercarbonsäure ohne spezielle Katalysatoren verringert.

EP-A-1354905 offenbart ein Verfahren zur Herstellung von Verbindungen der Formel (1) 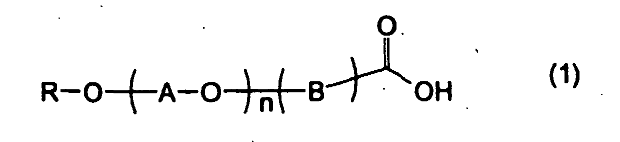 worin
- A: C₂- bis C₄-Alkylen,
- B: C₁- bis C₄-Alkylen
- n: eine Zahl von 1 bis 100, und
- R: C₁- bis C₃₀-Alkyl, C₂- bis C₃₀-Alkenyl, oder C₆- bis C₃₀-Aryl
bedeuten,
indem man ein basisches Gemisch von oxethylierten Alkoholen der Formel 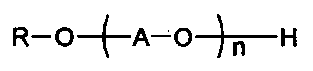 und deren Alkoholaten mit einer C₂- bis C₅-Chlorcarbonsäure alkyliert und das so erhaltene basische Zwischenprodukt nach Absäuerung durch Waschen mit wässriger Sulfatlösung so lange aufreinigt, bis die so erhaltene Ethercarbonsäure eine Leitfähigkeit von < 1000 µS/cm besitzt.

DE-C-10123210 offenbart Ethercarbonsäuren auf Basis von alkoxylierten 2-Mercaptobenzthiazolen, die eine gute Filmbildung und Filmpersistenz aufweisen und daher als korrosionsinhibierende Mittel für die Metallbearbeitung als auch für Erdöl- und Erdgasförderung und -verarbeitung Verwendung finden.

Die Verwendung von Ethercarbonsäuren als oberflächenaktive Additive hängt stark von deren Fähigkeit ab, neben guten Emulgatoreigenschaften und Kalkseifendispergiervermögen gleichzeitig ein hervorragendes Benetzungsverhalten, beispielsweise auf Faseroberflächen, zu besitzen und hinsichtlich Korrosionsschutz und Schmiereigenschaften Filme auf Metalloberflächen zu bilden, die auch bei starker mechanischer Belastung, wie beim Schleifen, Schneiden und Bohren von Metallwerkstücken bzw. bei hohen Fließgeschwindigkeiten und Drücken, persistent sind.

Diese Eigenschaften wurden hinreichend durch Ethercarbonsäuren des Standes der Technik auf Basis alkoxylierter Alkylphenole (APEs), vor allem alkoxylierter Nonylphenole (NPEs) erfüllt. Gemäß den europäischen Richtlinien 2003/53/EG und 76/769/EWG Anhang I Nr. 46 besitzen jedoch NPEs ein erhöhtes Gefährdungspotential für Mensch und Umwelt und dürfen daher in Konzentrationen über 0,1 Massen-% nicht mehr in Verkehr gebracht werden.

Es bestand daher die Aufgabe, neue, alternative Stoffe zu Ethercarbonsäuren auf Basis NPEs aufzufinden, die kein erhöhtes Gefährdungspotential besitzen und vergleichbare oder verbesserte oberflächenaktive Eigenschaften aufzeigen.

Überraschenderweise wurde gefunden, dass Ethercarbonsäuren auf Basis von alkoxylierten Styrylphenolen (1-Phenylethylphenole) ausgezeichnete Emulgatoreigenschaften mit hohem Kalkseifendispergiervermögen und eine exzellente Filmbildung besitzen sowie sehr gute ökotoxikologische Eigenschaften aufweisen.

Gegenstand der Erfindung sind daher Verbindungen der Formel (1) 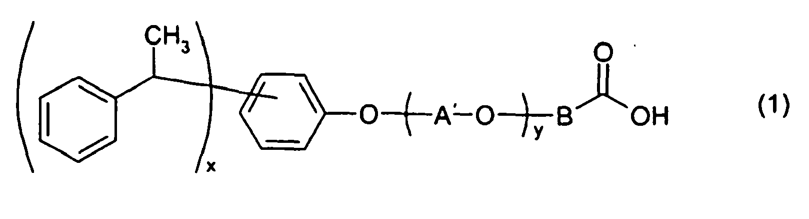 worin
- A: C₂- bis C₄-Alkylen,
- B: C₁- bis C₄-Alkylen,
- x: eine Zahl von 1 bis 3, und
- y: eine Zahl von 1 bis 100
bedeuten, als oberflächenaktiver Wirkstoff.

Ein weiterer Gegenstand der Erfindung ist die Verwendung der Verbindungen der Formel 1 als oberflächenaktiver Wirkstoff, in Metallbearbeitungsmitteln, gewerblichen Reinigern und Hilfsmitteln für die Textil-, Leder- und Papierverarbeitung.

Ein weiterer Gegenstand der Erfindung ist ein Verfahren zur Herstellung einer oberflächenaktiven Zusammensetzung, indem man zu einer Zusammensetzung ohne oberflächenaktive Eigenschaften die Verbindung der Formel 1 zugibt.

A bedeutet vorzugsweise Propylen oder Ethylen, insbesondere Ethylen. In einer weiteren bevorzugten Ausführungsform der Erfindung steht die Gruppe (A-O)_{y} für eine gemischte Alkoxygruppe, die Ethylen-, Propylen- und Butylenreste enthalten kann. Handelt es sch um eine gemischte Alkoxygruppe, so liegt das molare Verhältnis der vom Ethylenoxid abgeleiteten Gruppen zu den von Propylen- oder Butylenoxid abgeleiteten Gruppen vorzugsweise zwischen 10:1 und 1:1.

y steht vorzugsweise für eine Zahl zwischen 2 und 70, insbesondere 3 bis 50.

x steht vorzugsweise für 2 oder 3, insbesondere 3.

Ein weiterer Gegenstand der Erfindung ist die Verwendung der Salze der Verbindungen der Formel (1) entsprechend Formel (2) 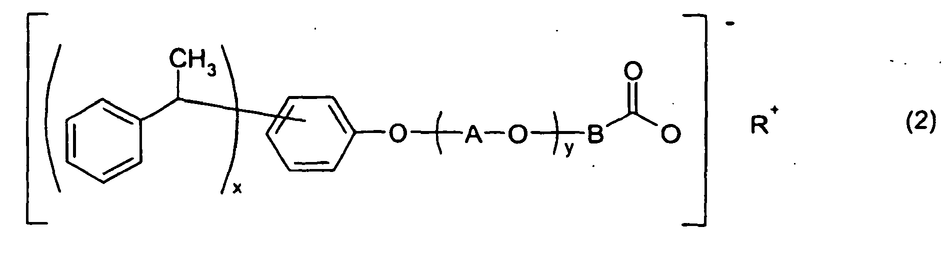 worin
- A: C₂- bis C₄-Alkylen,
- B: C₁- bis C₄-Alkylen,
- x: eine Zahl von 1 bis 3,
- y: eine Zahl von 1 bis 100, und
- R⁺: ein Kation
bedeuten, als oberflächenaktiver Wirkstoff.

Ein weiterer Gegenstand der Erfindung ist die Verwendung der Verbindungen der Formel 2 als oberflächenaktiver Wirkstoff, in Metallbearbeitungsmittel, gewerblichen Reinigern und Hilfsmittel für die Textil-, Leder- und Papierverarbeitung.

Ein weiterer Gegenstand der Erfindung ist ein Verfahren zur Herstellung einer oberflächenaktiven Zusammensetzung, indem man zu einer Zusammensetzung ohne oberflächenaktive Eigenschaften die Verbindung der Formel 2 zugibt.

A, B, x und y haben die bereits oben angegebenen Bedeutungen.

In einer bevorzugten Ausführungsform steht R für Alkali- oder Erdalkaliionen, insbesondere Lithium, Natrium, Kalium, Magnesium, oder Calcium.

In einer weiteren bevorzugten Ausführungsform werden als Kationen Ammoniumionen der Formel NR¹R²R³R⁴ verwendet, wobei R¹, R², R³ und R⁴ unabhängig voneinander H, C₁- bis C₂₂-Alkyl, C₆- bis C₁₈-Aryl, C₇- bis C₂₂-Alkylaryl und/oder C₁- bis C₂₂-Alkenyl bedeuten können. Die Reste R¹, R², R³ und R⁴ können Heteroatome wie N, P, O, S enthalten. Die Ammoniumreste können Monoalkylammonium-, Dialkylammonium-, Trialkylammonium- oder Tetraalkylammoniumreste sein, worin die Alkylsubstituenten unabhängig voneinander mit bis zu 3 Hydroxygruppen besetzt sein können. Vorzugsweise steht R für Ammoniumreste, die einen, zwei, drei oder vier C₂- bis C₁₀-Alkylreste tragen. In einer weiteren bevorzugten Ausführungsform können einer, zwei oder drei der Reste R¹ bis R⁴ alkoxyliert sein.

Geeignete Amine für die Herstellung von Ammoniumkationen R sind Monoamine mit primärer oder sekundärer Aminofunktion wie Methylamin, Ethylamin, Butylamin, Laurylamin, Cocosfettamin, Stearylamin, Dimethylamin, Diethylamin, Dibutylamin, aber auch Di- und Polyamine wie z.B. 3-Dimethylaminopropylamin, 3-Diethylaminopropylamin, 3-Morpholinopropylamin, Diethylentriamin, Triethylentetramin oder Tetraethylenpentamin.

Geeignete Aminoalkohole für die Herstellung von Ammoniumkationen R sind beispielsweise N,N-Dimethylaminoethanol, N,N-Diethylaminoethanol, N,N-Dibutylaminoethanol, 3-Dimethylaminopropanol, N-Hydroxyethylmorpholin, Monoethanolamin, Diethanolamin, Triethanolamin, 3-Aminopropanol, Isopropanolamin, 2(2-Aminoethoxy)ethanol und Cyclohexylamino- N,N-diethanol.

Geeignete Aminalkylthiole für die Herstellung von Ammoniumkationen R sind Cysteamin und Cystamin.

Die erfindungsgemäßen Verbindungen der Formel 1 können dadurch hergestellt werden, dass man Styrylphenole zunächst alkoxyliert und dann mit Monochlorcarbonsäuren umsetzt. Durch den Begriff Styrylphenol werden Mono-, Di- und Tristyrylphenole (Mono-, Di- und Tri(1-phenylethyl)phenole) und deren Mischungen umfasst.

Styrylphenole lassen sich gemäß des Standes der Technik durch Friedel-Crafts-Alkylierung von Phenol herstellen. Bevorzugte Substitution erfolgt in o- und p-Position.

Styrylphenol wird im allgemeinen mit Ethylenoxid, Propylenoxid, Butylenoxid oder Mischungen verschiedener solcher Alkylenoxide umgesetzt, wobei Ethylenoxid oder Mischungen aus Ethylenoxid und Propylenoxid bevorzugt sind. Bezogen auf Styrylphenol werden 1-100 Mol Alkylenoxid beaufschlagt, bevorzugt 2-70 Mol, besonders bevorzugt 3-50 Mol.

Die Alkoxylierung erfolgt im allgemeinen ohne die Verwendung von Lösemitteln. Werden Lösemittel verwendet, sind inerte Ether wie Dioxan, Tetrahydrofuran, Glyme, Diglyme und MPEGs bevorzugt. Wasser als auch Alkohole, wie Propanole, Butanole sowie oxethylierte Monoalkohole wie Butylglycol, Isobutylglycol und Butyldiglycol können zur Anwendung kommen, führen aber zu einem hohen Anteil an Nebenprodukten.

Als basische Verbindung zur Herstellung des oxethylierten Styrylphenols können Erdalkali-/ Alkalimetallhydroxide oder -alkoholate (Natriummethylat, Natriumethylat, Kalium-tert.-butylat) verwendet werden, bevorzugt sind aber Alkalimetallhydroxide, besonders Natriumhydroxid oder Kaliumhydroxid.

Die basischen Verbindungen werden in Mengen von ca. 5-95 Mol-% bezogen auf Styrylphenol eingesetzt, bevorzugt zwischen 15 und 90 Mol-%, besonders bevorzugt zwischen 20-60 Mol-%.

Ausgehend vom Styrylphenol werden das zur Oxalkylierung notwendige Phenolat durch Umsetzung mit den basischen Verbindungen hergestellt. Um höhere Anteile an Nebenprodukten (Glykole, Glykolether niederer Alkohole) im Endprodukt zu vermeiden, sollte das hierbei entstehende Reaktionswasser oder der entsprechende niedere Alkohol aus dem Reaktionsgemisch vor der Umsetzung mit dem Alkylenoxid entfernt werden. Dies kann entweder durch Umsetzung des Styrylphenols mit einem Alkalihydroxid und Abdestillieren des Reaktionswassers geschehen oder durch Umsetzung des Basisalkohols mit einem Alkoholat eines niederen Alkohols und Abdestillieren des niederen Alkohols. Andererseits kann Styrylphenol in einem Zweischrittverfahren, im ersten Schritt ohne Zugabe der basischen Verbindungen monoalkoxyliert werden. In einem weiteren Schritt erfolgt dann die notwendige Umsetzung zum Alkoholat.

Die erhaltene Mischung aus Styrylphenol und dem entsprechenden Styrylphenolat bzw. Styrylphenylalkoxylat wird dann mit ca. 1-100 mol eines Alkylenoxids, bevorzugt Ethylenoxid und/oder Propylenoxid umgesetzt, die Reaktionstemperaturen liegen hierbei bei ca. 80 bis 160°C. Dabei tritt bei einer mit höheren Alkalimengen katalysierten Reaktion eine engere Homologenverteilung ein.

Im anschließenden Reaktionsschritt wird die Styrylphenyl-Oxalkylat-Mischung mit einem Chlorcarbonsäurederivat und einer Base, bevorzugt trockenem Chloressigsäure-Natriumsalz und Natriumhydroxid umgesetzt. Dies kann geschehen, indem man die Oxalkylat-Mischung mit 100 bis 150 Mol-% Natriumchloracetat bei 30 bis 100°C umsetzt und gleichzeitig oder nacheinander mit festem Natriumhydroxid oder Kaliumhydroxid versetzt, so dass die Summe aus der in der Oxalkylatmischung bereits vorliegenden Base und der zusätzlich zugegebenen Basenmenge der Menge an Natriumchloracetat entspricht. Die aus der Umsetzung mit dem Alkylenoxid bereits enthaltene Basenmenge kann somit direkt für die anschließende Williamson-Synthese genutzt werden und muss nicht, wie bei der Synthese eines Standard-Oxalkylats, ausgewaschen werden.

Anschließend an die Alkylierungsreaktion kann die entstehende Lösung des Styrylphenyl-Ethercarbonsäure-Alkalisalzes entweder direkt als erfindungsgemäße Verbindung Verwendung finden oder in die freie Styrylphenyl-Ethercarbonsäure überführt werden. Hierzu wird mit starker Mineralsäure (Salzsäure, Schwefelsäure) auf pH < 3 angesäuert und die Styrylphenyl-Ethercarbonsäure durch Phasentrennung oberhalb ihres Trübungspunktes heiß als Oberphase abgetrennt.

Die erfindungsgemäßen, freien Ethercarbonsäuren lassen sich auch direkt durch Oxidation der Styrylphenyl-Oxalkylat-Mischung mit verschiedenen Reagenzien (Luftsauerstoff, Hypochlorit, Chlorit) unter Katalyse mit verschiedenen Katalysatoren herstellen. Besonders bevorzugt ist die Oxidation mittels Sauerstoff unter Verwendung von Platin-Trägerkatalysatoren.

Die Oxidation kann mit oder ohne Verwendung von Lösemitteln erfolgen. Die Oxidation erfolgt bei Temperaturen von 10 bis 250°C, bevorzugt bei 20 bis 150 °C, besonders bevorzugt bei 50 bis 100 °C.

Die Herstellung der erfindungsgemäßen Styrylphenyl-Ethercarbonsäureammoniumsalze erfolgt im allgemeinen durch direkte Umsetzung der freien Säure mit den entsprechend funktionalisierten Aminen bei Temperaturen unter 60°C.

### Beispiele:

### Beispiel 1 (Tristyrylphenol + 5 EO):

In einem 2 l Ethoxylierungsautoklaven wurden 812 g Tristyrylphenol unter Stickstoffspülung vorgelegt und unter NaOH Katalyse (1 %) bei 120 bis 130°C mit Ethylenoxid begast, bis 10 mol EO unter Druckkonstanz abreagiert waren. Es wurde 1 h bei 150°C nachreagiert. Nach Abdestillation leicht flüchtiger Komponenten wurde das Produkt als klare farblose Flüssigkeit erhalten. Nach OH-Zahl ergab sich ein durchschnittlicher EO-Gehalt von 4,5. Der Trübungspunkt wurde mit 23°C bestimmt.

### Beispiel 2 (Tristyrylphenol + 10 EO)

In einem 2 l Ethoxylierungsautoklaven wurden 609 g (1,5 mol) Tristyrylphenol unter Stickstoffspülung vorgelegt und unter NaOH Katalyse (1 %) bei 120 bis 130°C mit Ethylenoxid begast, bis 15 mol EO unter Druckkonstanz abreagiert waren. Es wurde 1 h bei 150°C nachreagiert. Nach Abdestillation leicht flüchtiger Komponenten wurde das Produkt als klare farblose Flüssigkeit erhalten. Nach OH-Zahl ergab sich ein durchschnittlicher EO-Gehalt von 10,5. Der Trübungspunkt wurde mit 68°C bestimmt.

### Beispiel 3 (Tristyrylphenol + 5 EO-ECS)

In einer 2 I Rührapparatur wurden 596 g Tristyrylphenol + 5 EO (1 mol entsprechend OH-Zahl) unter Stickstoffspülung vorgelegt und auf 40°C erwärmt. Dann wurden 140 g (1,2 mol) Natriumchloracetat eingetragen und die Reaktionsmischung auf 50°C erwärmt. Nach jeweils 30 min wurden 48 g (1,2 mol) NaOH-Microprills in 4 Portionen so zugegeben, dass die Temperatur 50-60°C nicht übersteigt. Es wurde 2 h bei 80-100°C nachreagiert. Danach wurde 440 g 10 % Salzsäure zulaufen lassen, die Mischung auf 95°C erhitzt und in eine beheizbare Rührapparatur mit Bodenablass überführt. Die Phasentrennung erfolgte nach 30 min bei 105°C, wobei 560 g wässrige Unterphase abgetrennt und 660 g Tristyrylphenol + 5 EO-ECS mit einem Wassergehalt von 4,5 % erhalten wurden.

### Beispiel 4 (Tristyrylphenol + 10 EO-ECS)

In einer 2 I Rührapparatur wurden 866 g Tristyrylphenol + 5 EO (1 mol entsprechend OH-Zahl) unter Stickstoffspülung vorgelegt und auf 40°C erwärmt. Dann wurden 140 g (1,2 mol) Natriumchloracetat eingetragen und die Reaktionsmischung auf 50°C erwärmt. Nach jeweils 30 min wurden 48 g (1,2 mol) NaOH-Microprills in 4 Portionen so zugegeben, dass die Temperatur 50-60°C nicht übersteigt. Es wurde 2 h bei 80-100°C nachreagiert. Danach wurde 440 g 10 % Salzsäure zulaufen lassen, die Mischung auf 95°C erhitzt und in eine beheizbare Rührapparatur mit Bodenablass überführt. Die Phasentrennung erfolgte nach 15 min bei 105°C, wobei 535 g wässrige Unterphase abgetrennt und 955 g Tristyrylphenol + 10 EO-ECS mit einem Wassergehalt von 6,5 % erhalten wurden.

Verwendung der erfindungsgemäßen Verbindungen als oberflächenaktives Additiv für wassermischbare Kühlschmiermittel, Reinigungsflüssigkeiten und Oberflächenbehandlungen.

Die erfindungsgemäßen Verbindungen können für wassermischbare Kühlschmiermittel, Reinigungsflüssigkeiten und Oberflächenbehandlungen in Form von milchig-opalen und semi-synthetischen (transparenten) Emulsionen bzw. voll-synthetischen (ölfreie) Lösungen verwendet werden. Die Einsatzkonzentrationen sind 1 bis 50 %, vorzugsweise 2 bis 10 %, besonders bevorzugt 3 bis 5 %.

Die erfindungsgemäßen Verbindungen können in beliebigen Verhältnissen mit Emulgatoren und Coemulgatoren (anionische, beispielsweise Sulphonate, Carbonsäuren, Ethercarbonsäuren); (nichtionische, beispielsweise Alkylalkoxylate, mono- und polyvalente Alkohole), Korrosionsinhibitoren (beispielsweise Alkenylbernsteinsäurederivate, Sulphonate, mono- und polyvalente Carbonsäuren, Ethercarbonsäuren, Fettsäureamide, Amine, heterocyclische Verbindungen und Borsäure), Schmiermitteln (beispielsweise native und synthetische Fette und Öle, Fettsäuren, Ester und Amide, Polymere), EP/AW-Additiven (extreme pressure/anti-wear = Hochdruck/Anti-Abrasion; beispielsweise Schwefelverbindungen, Phosphorsäureester, Dithiophosphate und Molybdänverbindungen), Bioziden (beispielsweise Borsäure, Formaldehyd-abgebende Stoffe und heterocyclische Verbindungen), Chelatbildnern und Sequestriermitteln (beispielsweise Alkanolaminen, Aminocarbonsäuren), Entschäumern (beispielsweise Silicon- und Fluorverbindungen), Antimisting-Additiven (= Nebelverhinderer) und Basisflüssigkeiten (beispielsweise Mineralöle, synthetische und native Ester und Polyester, Polyalkylenglykole sowie Wasser) eingesetzt werden.

Die Wirksamkeit der erfindungsgemäßen Verbindungen als Emulgator wurde anhand einer Richtformulierung für transparente Emulsionen geprüft (Konzentrat: 40 % Mineralöl, 40 % Korrosionsinhibitor-Package, 5 % Emulgatoren, 10 % Wasser und 5 % zu prüfende Ethercarbonsäure). Dabei wurde sowohl die Stabilität des Konzentrats (bei T = 0°C, 20° und 40°C) als auch die der Emulsion (5 % Konzentrat in DIN-Wasser 20°dH) nach Herstellung und nach 24 h visuell beurteilt. Die erfindungsgemäßen Verbindungen ergaben stabile Konzentrate und Emulsionen.
Die Korrosionsschutzprüfung erfolgte in Anlehnung an die DIN-Norm 51360, Teil 2 (Filterpapiertest) und dient der Beurteilung der Korrosion von Eisenmetall. Als Maß für die Korrosion dienen Art und Anzahl der Korrosionsabzeichnungen auf einem Rundfilter, die durch Einwirken eines mit Wasser gemischten Kühlschmierstoffes (KSS) auf genormte Graugussspäne (Spangröße: 3 bis 6 mm²) entstehen. Die Beurteilung erfolgt durch Sichtprüfung und Einstufung des Korrosionsgrades (1 bis 4) nach Vergleichstabelle.

Als Vergleich wurden kommerziell erhältliche Emulgatoren (Emulsogen® COL 050 und COL 100) mit vergleichbarem Alkoxylierungsgrad herangezogen. Es handelt sich dabei im wesentlichen um Ethercarbonsäuren der Zusammensetzung Oleyl-O-(EO)₅-CH₂-COOH (Emulsogen COL 050) bzw. das Homologe mit 10 EO-Gruppen (EmulsogenCOL 100).

Die geprüften Additive wurden mit Triethanolamin (TEA) unter Bildung des entsprechenden Ammoniumsalzes auf pH 8,9 eingestellt.

**Tabelle 1: Korrosionsschutzprüfung nach DIN (Filterpapiertest)**

| Beispiel | Emulgator/Korrosionsinhibitor | Konzentration des Korrosionsinhibitors | | |
|---|---|---|---|---|
| | | 2% | 3% | 4% |
| 5 (V) | Emulsogen COL 050 | 1-2 | 0-1 | 0 |
| 6 (V) | Emulsogen COL 100 | 4 | 3 | 2-3 |
| 7 | aus Beispiel 3 | 2 | 0-1 | 0 |
| 8 | aus Beispiel 4 | 3 | 1 | 1 |

Die Prüfung des Kalkseifendispergiervermögens der erfindungsgemäßen Verbindungen erfolgte in Anlehnung an die DIN-Norm 53903 und dient der Beurteilung der Fähigkeit, die durch die Härtebildnern des Wassers verursachten Ausfällungen (Kalkseifen) in Lösung zu halten. Als Maß für das Kalkseifendispergiervermögen dient der K-Wert, der das Verhältnis von dispergierter Menge Kalksseife, berechnet als 100 % Natriumoleat, je 1 g Emulgator angibt. Als Vergleich wurden ebenfalls die o.g. Emulgatoren (Beispiel 5 und 6) herangezogen.

**Tabelle 2: Kalkseifendispergiervermögen nach DIN**

| Beispiel | Emulgator/Korrosionsinhibitor | K-Wert |
|---|---|---|
| 9 (V) | Emulsogen COL 050 | 17 |
| 10 (V) | Emulsogen COL 100 | 33 |
| 11 | aus Beispiel 3 | 11 |
| 12 | aus Beispiel 4 | 20 |

## Patentansprüche

1. Verwendung von Verbindungen der Formel (1) 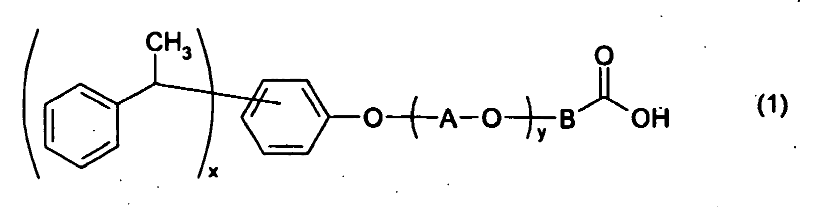 worin
A C₂- bis C₄-Alkylen,
B C₁- bis C₄-Alkylen,
x eine Zahl von 1 bis 3, und
y eine Zahl von 1 bis 100
bedeuten, als oberflächenaktiver Wirkstoff.

2. Verwendung gemäß Anspruch 1, worin A Propylen oder Ethylen bedeutet.

3. Verwendung gemäß Anspruch 1 und/oder 2, worin die Gruppe (A-O)_{y} für eine gemischte Alkoxygruppe, die Ethylen-, Propylen- und Butylenreste enthalten kann, steht.

4. Verwendung gemäß einem oder mehreren der Ansprüche 1 bis 3, worin die Gruppe (A-O)_{y} für eine gemischte Alkoxygruppe, die Ethylen-, Propylen- und Butylenreste enthält, steht, und worin das molare Verhältnis der vom Ethylenoxid abgeleiteten Gruppen zu den von Propylen- oder Butylenoxid abgeleiteten Gruppen zwischen 10:1 und 1:1 liegt.

5. Verwendung gemäß einem oder mehreren der Ansprüche 1 bis 4, worin y für eine Zahl zwischen 2 und 70 steht.

6. Verwendung gemäß einem oder mehreren der Ansprüche 1 bis 5, worin x für 2 oder 3 steht.

7. Verwendung gemäß einem oder mehreren der Ansprüche 1 bis 6, worin Salze der Verbindungen der Formel (1) gemäß Formel (2) 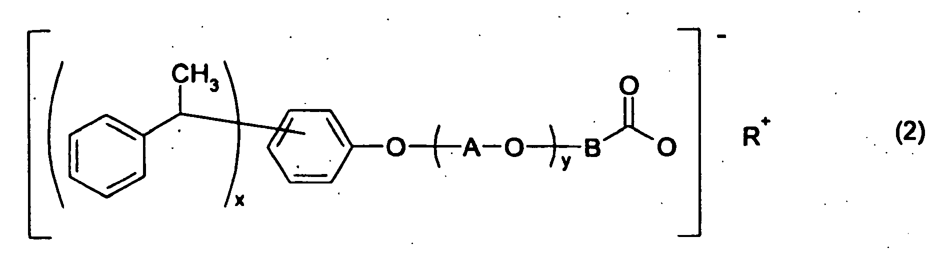 worin A, B, x und y die in einem oder mehreren der Ansprüche 1 bis 6 angegebene Bedeutung haben, und R ein Kation bedeutet, zur Anwendung kommen.

8. Verwendung gemäß Anspruch 7, worin R für Alkali- oder Erdalkaliionen, insbesondere Lithium, Natrium. Kalium, Magnesium, oder Calcium steht.

9. Verwendung gemäß Anspruch 7 und/oder 8, worin R für Ammoniumionen der Formel NR¹R²R³R⁴ steht, wobei R¹, R², R³ und R⁴ unabhängig voneinander H, C₁-bis C₂₂-Alkyl, C₆- bis C₁₈-Aryl, C₇- bis C₂₂-Alkylaryl und/oder C₁- bis C₂₂-Alkenyl bedeuten, die Heteroatome wie N, P, O, S enthalten können.

10. Verwendung gemäß einem oder mehreren der Ansprüche 1 bis 9 als oberflächenaktiver Wirkstoff in Metallbearbeitungsmitteln, gewerblichen Reinigern und Hilfsmitteln für die Textil-, Leder- und Papierverarbeitung.

## Claims

1. The use of compounds of the formula (1) 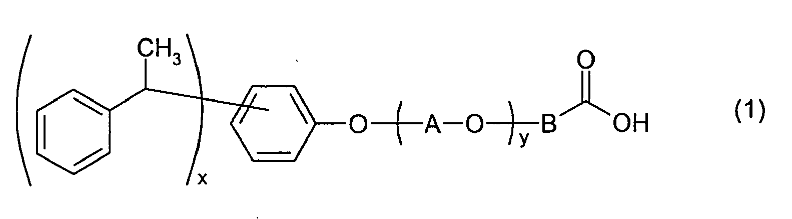 in which
A is C₂- to C₄-alkylene,
B is C₁- to C₄-alkylene,
x is a number from 1 to 3, and
y is a number from 1 to 100,
as surface-active ingredient.

2. The use as claimed in claim 1, in which A is propylene or ethylene.

3. The use as claimed in claim 1 and/or 2, in which the group (A-O)_{y} is a mixed alkoxy group which can contain ethylene, propylene and butylene radicals.

4. The use as claimed in one or more of claims 1 to 3, in which the group (A-O)_{y} is a mixed alkoxy group which contains ethylene, propylene and butylene radicals, and in which the molar ratio of the groups derived from ethylene oxide to the groups derived from propylene oxide or butylene oxide is between 10:1 and 1:1.

5. The use as claimed in one or more of claims 1 to 4, in which y is a number between 2 and 70.

6. The use as claimed in one or more of claims 1 to 5, in which x is 2 or 3.

7. The use as claimed in one or more of claims 1 to 6, in which salts of the compounds of the formula (1) according to formula (2) 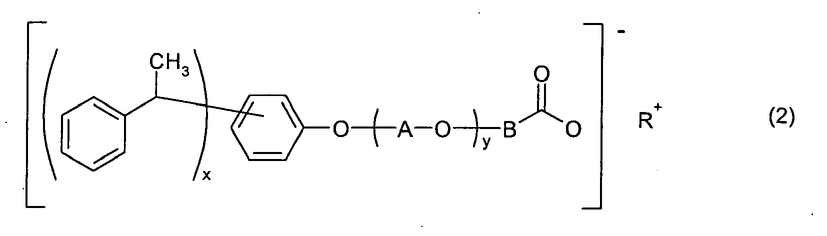 in which A, B, x and y have the meanings given in one or more of claims 1 to 6, and R is a cation, are used

8. The use as claimed in claim 7, in which R is alkali metal or alkaline earth metal ions, in particular lithium, sodium, potassium, magnesium or calcium.

9. The use as claimed in claim 7 and/or 8, in which R is ammonium ions of the formula NR¹R²R³R⁴, where R¹, R², R³ and R⁴, independently of one another, are H, C₁- to C₂₂-alkyl, C₆- to C₁₈-aryl, C₇- to C₂₂-alkylaryl and/or C₁- to C₂₂-alkenyl, which may contain heteroatoms such as N, P, O, S.

10. The use as claimed in one or more of claims 1 to 9 as surfactant in metal working compositions, industrial cleaners and auxiliaries for textile, leather and paper processing.

## Revendications

1. Utilisation de composés de formule (1) 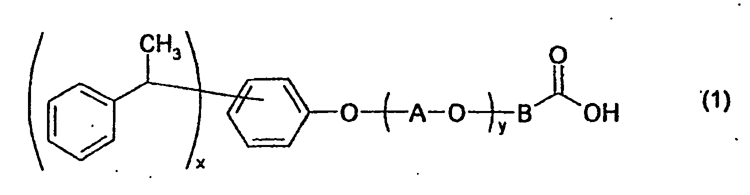 dans laquelle
A représente un groupe alkylène en C₂ à C₄,
B représente un groupe alkylène en C₁ à C₄,
x représente un nombre de 1 à 3, et
y représente un nombre de 1 à 100,
en tant que substance tensio-active.

2. Utilisation selon la revendication 1, dans laquelle A représente le propylène ou l'éthylène.

3. Utilisation selon la revendication 1 et/ou 2, dans laquelle le groupe (A-O)_{y} représente un groupe alcoxy mixte, qui peut contenir des groupes éthylène, propylène et butylène.

4. Utilisation selon l'une quelconque ou plusieurs des revendications 1 à 3, dans laquelle le groupe (A-O)_{y} représente un groupe alcoxy mixte, qui peut contenir des groupes éthylène, propylène et butylène, et dans laquelle le rapport molaire des groupes dérivés d'oxyde d'éthylène aux groupes dérivés d'oxyde de propylène ou d'oxyde de butylène est compris entre 10:1 1 et 1:1.

5. Utilisation selon l'une quelconque ou plusieurs des revendications 1 à 4, dans laquelle y représente un nombre compris entre 2 et 70.

6. Utilisation selon l'une quelconque ou plusieurs des revendications 1 à 5, dans laquelle x vaut 2 ou 3.

7. Utilisation selon l'une quelconque ou plusieurs des revendications 1 à 6, dans laquelle les sels des composés de formule (1) selon la formule (2) 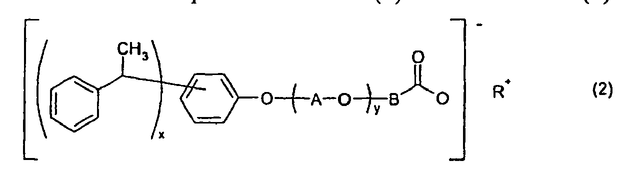 dans laquelle A, B, x et y ont la signification indiquée dans l'une quelconque ou plusieurs des revendications 1 à 6 et R représente un cation, sont utilisés.

8. Utilisation selon la revendication 7, dans laquelle R représente des ions alcalins ou alcalino-terreux, en particulier lithium, sodium, potassium, magnésium ou calcium.

9. Utilisation selon la revendication 7 et/ou 8, dans laquelle R représente des ions ammonium de formule NR¹R²R³R⁴, où R¹, R², R³ et R⁴ représentent, indépendamment les uns des autres, un atome d'hydrogène, un groupe alkyle en C₁ à C₂₂, aryle en C₆ à C₁₈, alkylaryle en C₇ à C₂₂ et/ou alcényle en C₁ à C₂₂, qui peuvent contenir des hétéroatomes tels que N, P, O, S.

10. Utilisation selon l'une quelconque ou plusieurs des revendications 1 à 9 en tant que substance tensio-active dans des produits pour la transformation de métaux, nettoyants industriels et adjuvants pour le traitement des textiles, du cuir et du papier.
